# EUROPEAN PATENT APPLICATION

(11) **EP 1 633 146 A1**
(43) Date of publication of application: **08.03.2006**
(21) Application number: 03768223.4
(22) Date of filing: 25.12.2003
(51) Int. Cl.: H04N 7/18

(54) **NURSE WORK SUPPORT SYSTEM**

(30) Priority: 06.06.2003 JP 2003162841
(71) Applicant: Olympus Corporation, Shibuya-ku, Tokyo (JP)
(72) Inventor: NOGUCHI, Toshiaki, Tachikawa-shi, Tokyo 190-0002 (JP); KUROSHIMA, Hisashi, Hachioji-shi, Tokyo 193-0832 (JP); SUZUKI, Eiri, Sagamihara-shi, Kanagawa 229-0038 (JP); HASEGAWA, Hitoshi, Yokohama-shi, Kanagawa 222-0002 (JP); GOCHO, Masanori, Hachioji-shi, Tokyo 192-0045 (JP)
(74) Representative: von Hellfeld, Axel
(86) International application number: PCT/JP2003/016690
(87) International publication number: WO 2004/110066

(57) **Abstract**

A plurality of portable terminals 11 carried by nurses and a plurality of TV cameras 8 disposed in respective rooms are able to send and receive images to and from a TV camera controller 9, by means of a wireless LAN, and hence a nurse can observe the status in each room, in real time, by means of his or her portable terminal 11. Moreover, the TV camera controller 9 is able to provide a multiple display of the images from the plurality of TV cameras 8, on a TV monitor 10, and therefore the status of each of the rooms can be monitored in real time in the reception area 1 also. Consequently, work can be carried out more effectively and at lower cost, in terms of the movements performed by the nurses.

## Description

### Technical Field

The present invention relates to a nursing work support system for supporting the work of nurses employed in medical examination work and required to move between various medical work areas.

### Background Art

In a hospital, endoscopic examination is constituted by a variety of interrelated tasks centred on the patient, which are carried out by staff in different posts.

A patient completes a consultation by following a series of steps, from initial procedures at reception, through preliminary treatment, endoscopic examination, and recovery, to the discharge of the patient.

Furthermore, the doctor is not only occupied with the actual examination, but must also undertake a combination of different tasks, such as treatment procedures, discussion with the patient, giving instructions to the nursing staff, and the like.

Conventionally, nurses move around the hospital following the various stages of the patient's treatment, thus providing support to the patient, and whilst performing various tasks relating to the patient, such as preparation of consultation materials and instruments, assistance during the consultation, clearing up of the materials and instruments, and the like, they must also perform other tasks, such as consultation support tasks, and the like.

In the prior art, Japanese Patent Laid-open No. 2002-101216 discloses a nurse call system which uses radio means. This application describes a system in which nurses are paged by transmitting the data relating to signals from nurse call machines situated in respective wards, to subsidiary radio devices, by referring to a call indicator table, and furthermore a nurse is able to speak with a patient in a ward and to provide appropriate treatment for the patient.

According to this prior art example, the nurse carries out appropriate treatment in response to a call made by a patient, but it is difficult for the nurse to predict the behaviour and state of the patients in advance and to organise his or her own tasks appropriately.

Moreover, in Japanese Patent Application No. 2002-252063, the present applicants proposed a hospital information system for assisting the medical actions of nursing staff. This application (Japanese Patent Application No. 2002-252063) provides a hospital information system which is able to assist the smooth implementation of scheduled medical actions by referring freely to the work schedule for medical actions. In terms of practical composition, a stand-alone PC terminal used for registering orders, and the like, is connected by a LAN to portable PDAs, Personal Digital Assistant: PDA, which are capable of inputting and outputting data, and is also connected to an internal hospital information management system which manages internal hospital information, and by means of the nurses each carrying a PDA, they are able freely to reference a work schedule list comprising a schedule of medical actions that must be carried out, confirm the medical actions in the work schedule whilst at the patient's bedside, where the actions are actually to be carried out, and then carry out the required medical actions accurately and correctly, thereby making it possible for a large number of tasks in a work schedule to be carried out smoothly.

However, currently, in seeking to achieve efficient management in hospitals, there is particular demand for major improvements in the work flow of tasks performed by nurses, and although there has been significant improvement in particular medical devices and various IT equipment has been introduced, as described in the prior art, progress has not yet been made in resolving the problem of unnecessary movements made by staff (ultimately, nurses have to move around the site, or move to a place where a PC (terminal) is located and operate that terminal, or operate a PDA to confirm their work details.)

Moreover, as described above, many nursing work support systems for endoscopic examination have been proposed, and these systems are principally aimed at increasing the efficiency of filing of endoscopic images, increasing the efficiency of reporting by adopting the use of electronic patient files, and increasing work efficiency by introducing unified management of patient information and examination reservation information.

These systems are based on PCs or PDAs, and in order to operate them, it is necessary either go to a place where PC is located, or to transmit various types of information by means of the limited functions of a PDA device. Therefore, these systems have not been easy to use.

Problems associated with using PCs include, for instance, "having to provide a location for installing PCs" and "restricting the locations where the system can be operated", and the like. Furthermore, if PDAs are employed, they use text information based on data handled by current PCs, and hence they still involve the inconvenience of operating a PC (if a nurse is busy, then the operation of sending and receiving data increases the workload for that nurse).

Moreover, the work efficiency of a nurse is not necessarily improved in the way described above (in particular, there is no improvement in the work flow in terms of the nurse having to move outside the endoscopic examination room).

Furthermore, in terms of equipment, the systems described above require large-scale equipment installation and construction works, and hence costly equipment investment is required.

The present invention was devised with the foregoing in view, an object thereof being to provide an endoscopic examination work support system capable of improving work in terms of the movement of nurses, in particular, effectively and inexpensively.

### Disclosure of Invention

The nursing work support system according to the present invention is a nursing work support system for supporting the work of nurses employed in medical examinations, who move between a plurality of medical work areas, comprising:
information obtaining means for obtaining area information relating to the plurality of medical work areas; and
portable terminal means carried by a nurse for communicating with the information obtaining means and acquiring area information relating to the plurality of medical work areas obtained by the information obtaining means.

Other features and advantages of the present invention will become sufficiently apparent from the following description of the invention.

### Brief Description of the Drawings

Fig. 1 to Fig. 14 relate to a first embodiment of the present invention;
Fig. 1 is a diagram showing the layout of a hospital for describing a nursing support system;
Fig. 2 is a diagram showing a portable terminal carried by a nurse working in the hospital shown in Fig. 1;
Fig. 3 is a diagram showing a cradle to which the portable terminal in Fig. 2 can be connected;
Fig. 4 is a diagram for describing calls between a plurality of portable terminals as illustrated in Fig. 2;
Fig. 5 is a diagram for describing an external device which can be connected to the external device connector of the portable terminal illustrated in Fig. 2;
Fig. 6 is a diagram for describing a remote control function of the portable terminal illustrated in Fig. 2;
Fig. 7 is a diagram showing a modification example of the portable terminal in Fig. 2;
Fig. 8 is a diagram showing the composition of nursing support system illustrated in Fig. 1;
Fig. 9 is a first diagram for describing the action of the nursing support system illustrated in Fig. 1;
Fig. 10 is a second diagram for describing the action of the nursing support system illustrated in Fig. 1;
Fig. 11 is a third diagram for describing the action of the nursing support system illustrated in Fig. 1;
Fig. 12 is a fourth diagram for describing the action of the nursing support system illustrated in Fig. 1;
Fig. 13 is a fifth diagram for describing the action of the nursing support system illustrated in Fig. 1;
Fig. 14 is a sixth diagram for describing the action of the nursing support system illustrated in Fig. 1;
Fig. 15 and Fig. 16 relate to a second embodiment of the present invention;

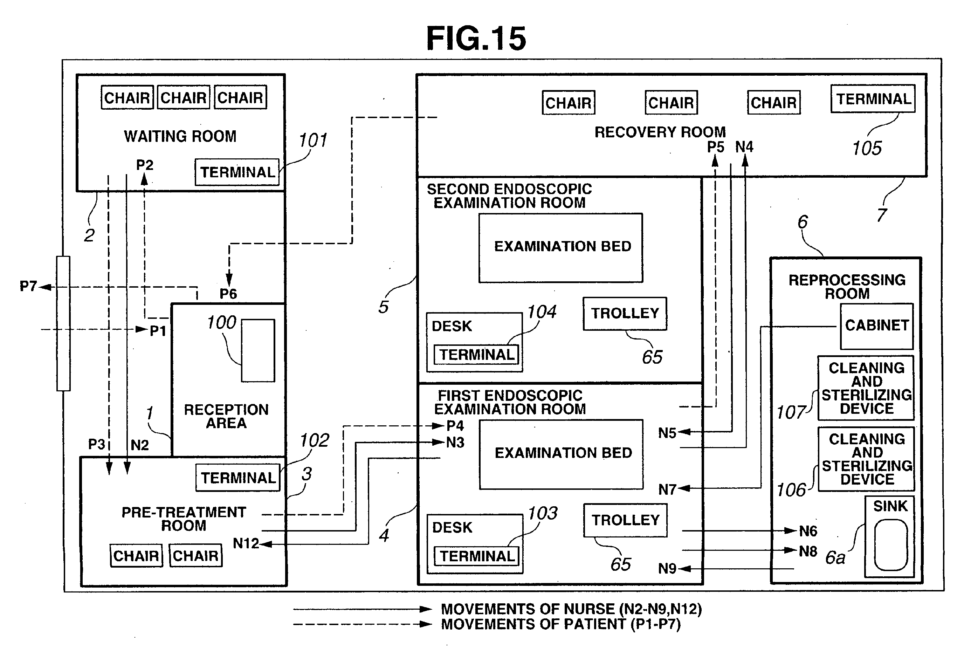

Fig. 15 is a diagram showing the layout of a hospital for describing a nursing support system;

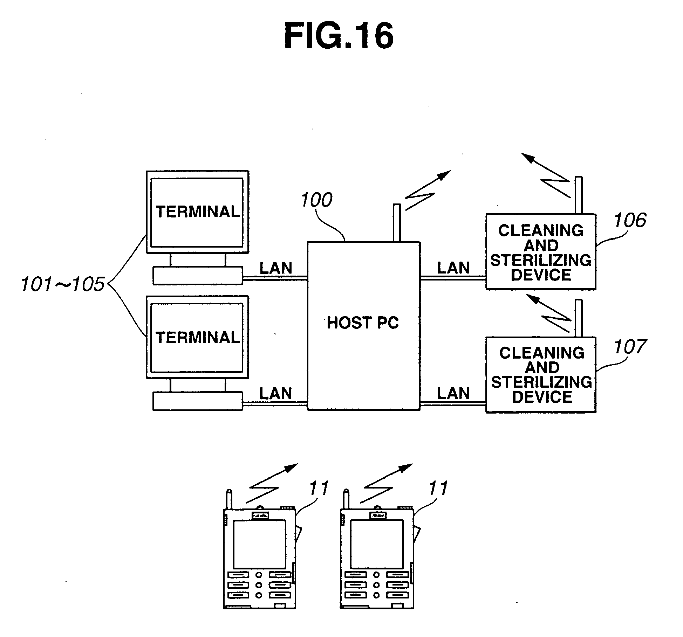

Fig. 16 is a diagram showing the composition of the nursing support system illustrated in Fig. 15;

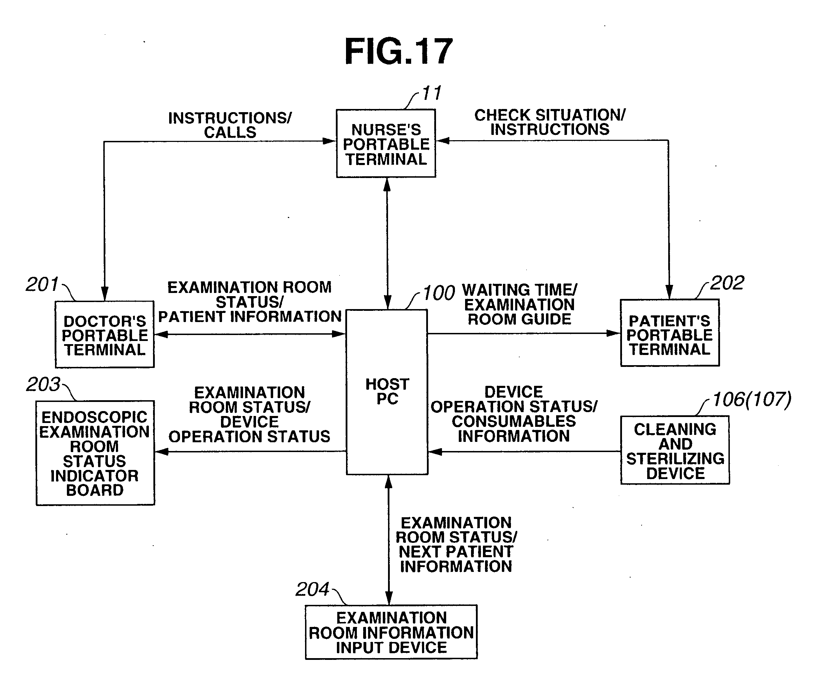

Fig. 17 to Fig. 20 relate to a third embodiment of the present invention;
Fig. 17 is a diagram showing the composition of a nursing support system;

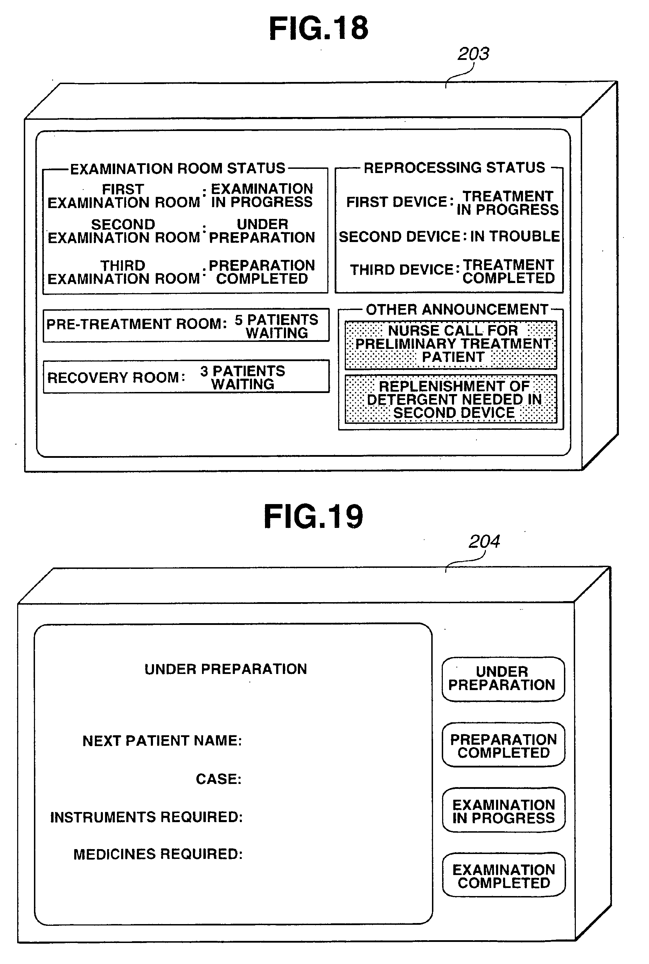

Fig. 18 is a diagram showing an endoscopic examination room status indicator board in Fig. 17;
Fig. 19 is a diagram showing an examination room information input device as illustrated in Fig. 17; and

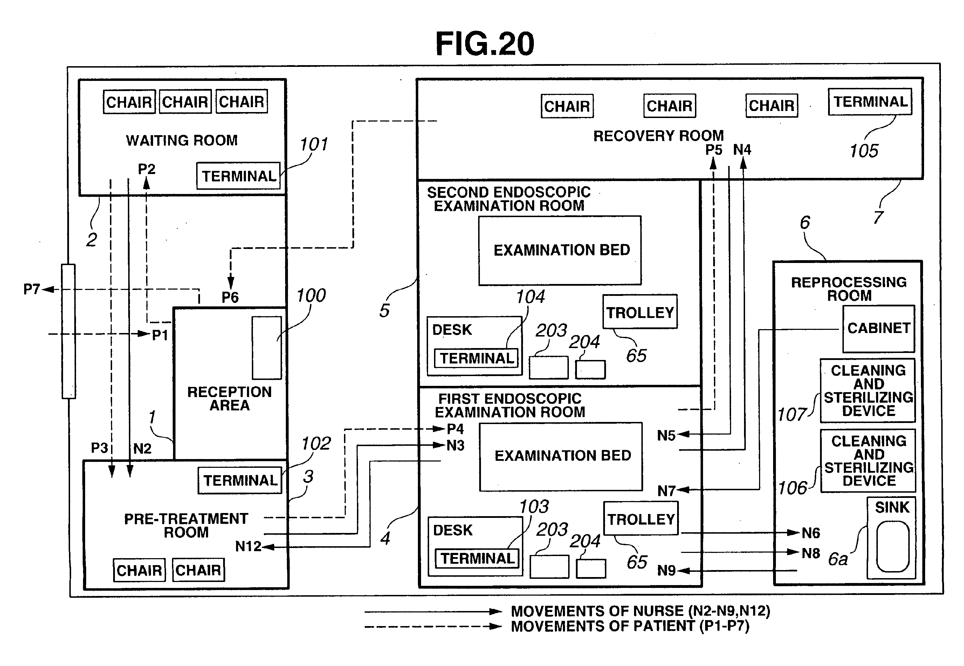

Fig. 20 is a diagram showing the layout of a hospital having the nursing support system illustrated in Fig. 17.

### Best Mode for Carrying Out the Invention

The present invention is described in detail below, with reference to the accompanying drawings.

### First Embodiment

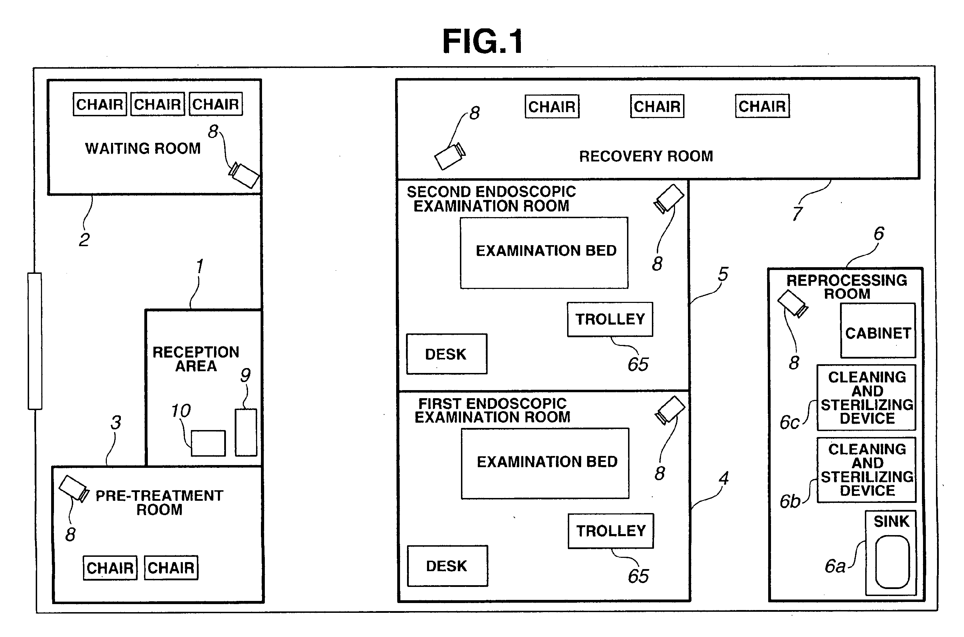

A nurse support system according to the present embodiment is now described, taking as an example a hospital which carries out endoscopic examinations, as illustrated in Fig. 1. The hospital in Fig. 1 has a layout comprising: a reception area 1 wherein patients are received; a waiting room 2 wherein patients await treatment; a pre-treatment room 3 wherein a patient receives preliminary treatment before examination; a plurality of endoscopic examination rooms 4, 5 where endoscopic examination is carried out; a reprocessing room 6 where the video endoscopes and other tools used in the endoscopic examinations are cleaned and sterilised; and a recovery room 7 where patients can rest and recover after examination has been completed.

Television (TV) cameras 8 capturing images of the interior of the rooms are installed respectively in the waiting room 2, the pre-treatment room 3, the endoscopic examination rooms 4, 5, the reprocessing room 6 and the recovery room 7. Moreover, a TV camera controller 9 for controlling the TV cameras 8 and a TV monitor 10 for providing a multiple display of the plurality of camera images captured by the TV cameras 8 are provided in the reception area 1.

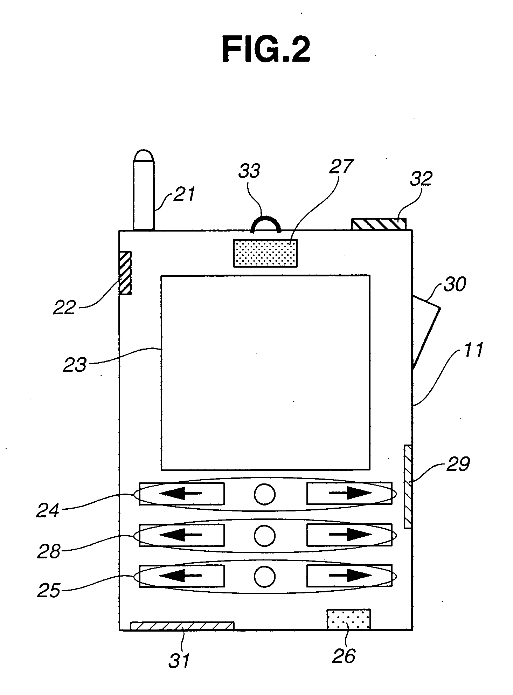

Meanwhile, in the present embodiment, a nurse working in this hospital performs his or her work whilst carrying a portable terminal 11 such as that illustrated in Fig. 2. A portable terminal 11 is carried, for example, by each one of the nurses working in the hospital (if necessary, one may also be carried by each of the reception staff, or doctors).

The portable terminal 11 comprises: a voice communications function for processing voice signals from the TV camera controller 9, and performing two-way communications; an image processing section (image communications function) for receiving camera image from the TV camera controller 9 and displaying them by means of a display function of the portable terminal 11; a data sending and receiving function for performing data communications with a medical information management system (not illustrated); a display function for displaying the camera images and data sent by the medical information management system, and the like; an external device connection function for connecting an external device, providing expansion functions, for example, a hands-free set for voice communications, or a head mounted display whereby the camera images, or data sent by the medical information management system, or the like, is displayed directly in front of the nurse's eyes; and an ID recognition function for recognising and storing the ID of the operator's (for example, the nurse's) portable terminal 11 (by means of an ID memory card, for example); and the like. Moreover, the portable terminal 11 also comprises a data recognition function (for example, an RF-ID reader), which can link to a hospital logistical supply management system (not illustrated) that supports stock management, logistical flow management and ordering management, and the like, for consumables used in the hospital, and the like, as well as a remote control function for controlling the brightness of the lighting apparatus in the endoscopic examination rooms 4, 5, and switching the display of images from the endoscope, and the like.

In practical terms, the portable terminal 11 comprises: an antenna 21 for sending and receiving voice data and an antenna 22 for data communications (for example, LAN communications); an LCD monitor 23 for displaying camera images and data sent by the medical information management system, and the like; a camera image switch 24 for controlling the display mode of the camera images (for switching the TV camera images); a voice communications switch 25 for controlling the voice communications (switching the other party to converse with, or the like); a microphone 26 and speaker 27 used for voice communications; various data download switches 28 for controlling operations used to download data and display options during data communications; an external device connector 29 for connecting an external device; a talk switch 30 for controlling voice communications (for instance, switching between talk and receive modes); an ID recognition card memory slot reader 31 for recognising the ID of the portable terminal 11; an RF-ID reader 32 for linking to the hospital logistical supply management system; and a strap hook 33 for fitting a strap when carrying the portable terminal 11 around.

In Fig. 2, a composition wherein all of these functions are provided is illustrated, but it is also possible to provide only selected functions of these.

The RF-ID reader 32 is able to read in RF-ID tag information attached to data, such as the gauze used in an endoscopic examination, the (disposable) treatment tools, various medicines, and detergent used, and the like, and send this data for use by the internal stock management system or ordering system (not illustrated).

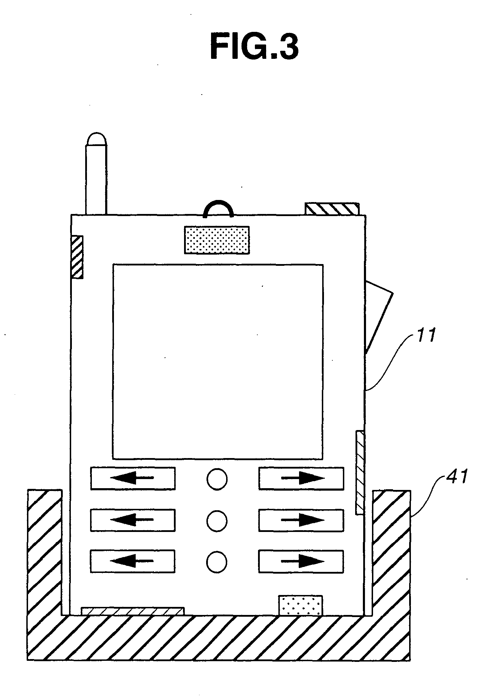

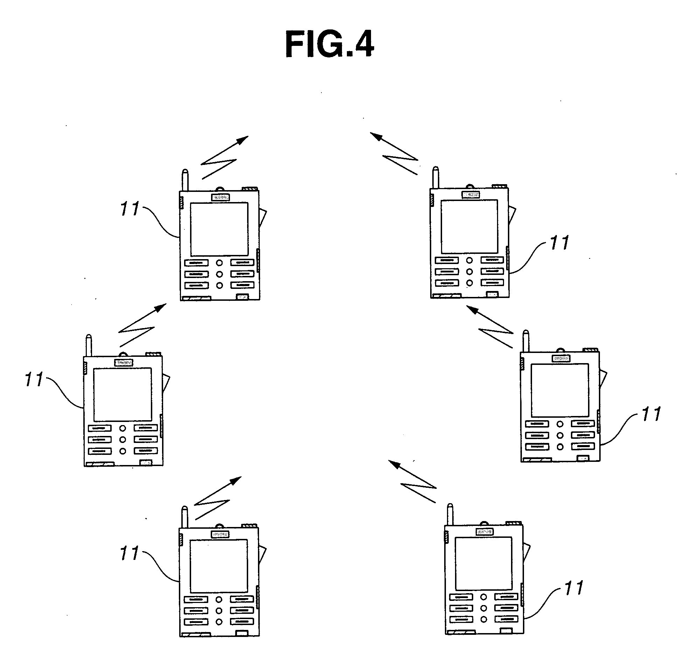

Furthermore, as shown in Fig. 3, the portable terminal 11 can be connected to a cradle 41 for sending and receiving data to and from a terminal PC (not illustrated), or the like, situated in reception 2, and moreover, as shown in Fig. 4, the portable terminal 11 also has a call function which allows calls to take place between a plurality of portable terminals.

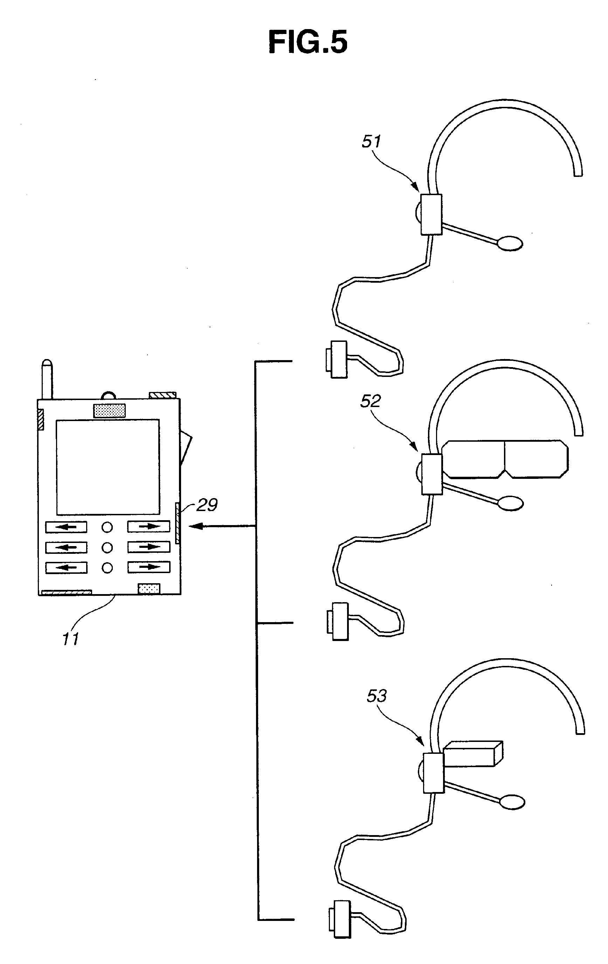

As shown in Fig. 5, a hands free call unit 51, a binocular type FMD unit 52, or a monocular type FMD unit 53 can be connected selectively to the external device connector 29 of the portable terminal 11, in such a manner that a nurse who is heavily occupied can use both hands to the full, and can check the screen display and perform voice communications without using his or her hands, thus reducing the work burden to some degree.

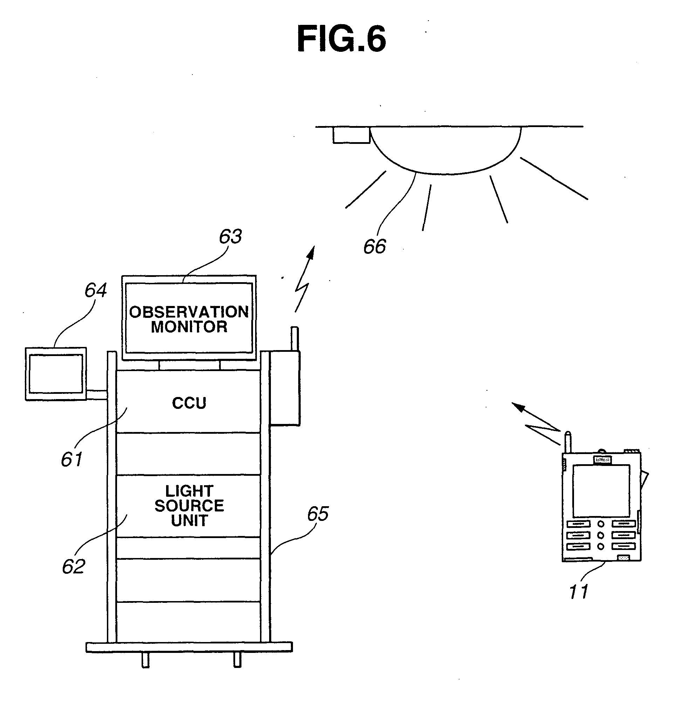

Here, a remote control function provided in the portable terminal 11 will be described. As shown in Fig. 6, in the endoscopic examination room 4, for example, a CCU (camera control unit) 61 for controlling the image provided by a video endoscope, a light source unit 62 for supplying illumination light to the video endoscope, an endoscopic examination monitor 63 for displaying images from the video endoscope, and a patient monitor 64 for showing the images from the video endoscope to the patient, are mounted on a patient trolley 65, and examination is carried out.

In this composition, the patient monitor 64 is provided for use when the endoscopic image is to be shown to the patient, in order that he or she can better understand the diagnosis results in the case of a normal endoscopic examination. Since the patient monitor 64 only shows images to the patient when necessary, the nurse can change position during the examination, upon the instructions of the doctor, and switch the display of the patient monitor 64 on and off, appropriately.

In order to remove the labour and lost time involved in these movements, a remote control function for controlling the on and off operation of the patient monitor 64 is provided in the portable terminal 11. Therefore, the nurse can switch the patient monitor 64 on and off without moving position during the examination.

Moreover, by utilising the remote control function for switching the patient monitor 64 on and off, the portable terminal 11 is also provided with a remote control function for switching the illumination apparatus 66 in the endoscopic examination room on and off.

Therefore, by using remote control functions of the portable terminal 11 of this kind, it is possible to reduce the work load borne by the nurse, and hence the nurse can carry out his or her work more efficiently.

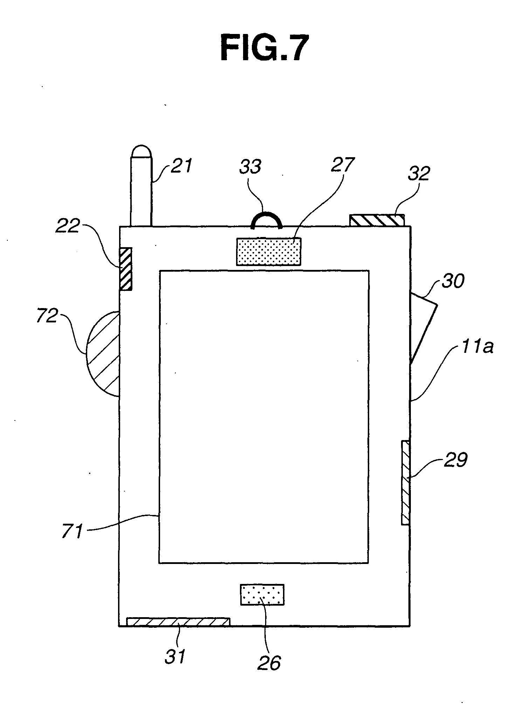

The portable terminal 11 is not limited to the composition shown in Fig. 2, and a portable terminal 11a using a touch panel type LCD monitor 71 as shown in Fig. 7 may also be used. By employing a touch panel type LCD monitor 71 of this kind, the various operating switches can be eliminated and hence the cost of the portable terminal 11a can be reduced, whilst by being able to make the LCD monitor 71 larger in size, a benefit is obtained in that the displays become easier to see. Moreover, for users who have difficulties in operating a touch panel screen, it is beneficial if a jog dial 72 is provided on the side of the main unit, whereby all of the operations can be carried out using one hand only, and the functions of the respective operating switches can be performed.

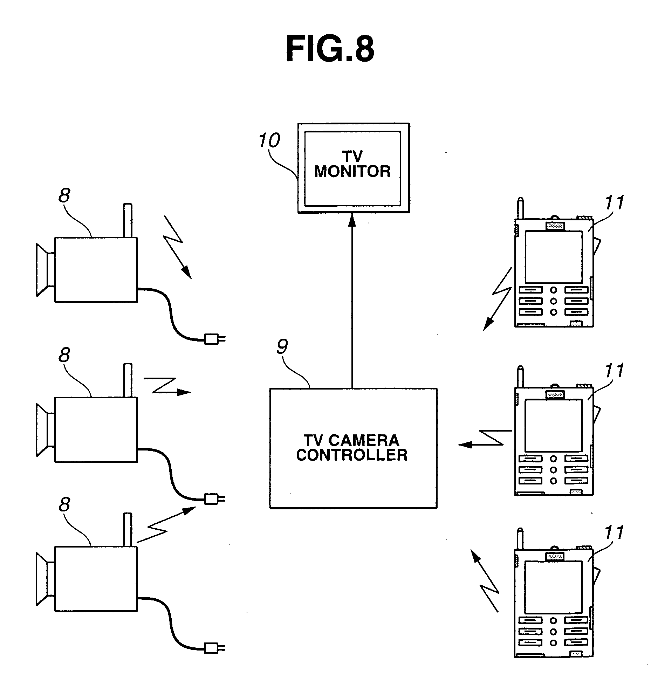

As shown in Fig. 8, in the present embodiment, a plurality of portable terminals 11 carried by nurses and a plurality of TV cameras 8 situated in respective rooms are able to communicate images with a TV camera controller 9, by means of a radio LAN, thereby making it possible for each nurse to observe the situation in each room, in real time, by means of the portable terminal 11. Furthermore, the TV camera controller 9 is able to provide a multiple display of images from a plurality of TV cameras 8 on the TV monitor 10, in such a manner that in the reception area 1, the situation in each of the rooms can be monitored in real time.

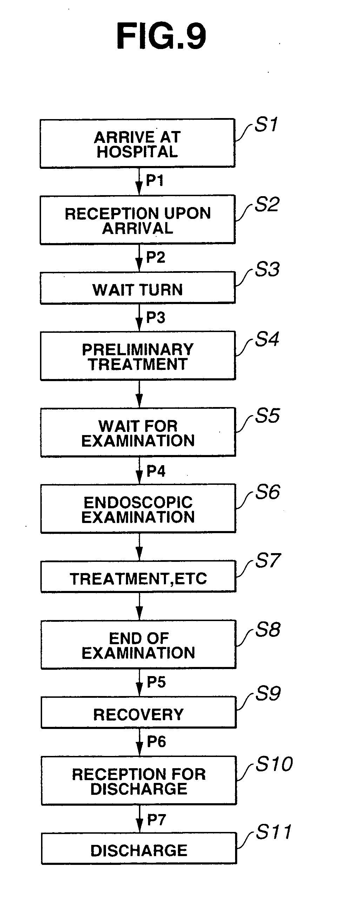

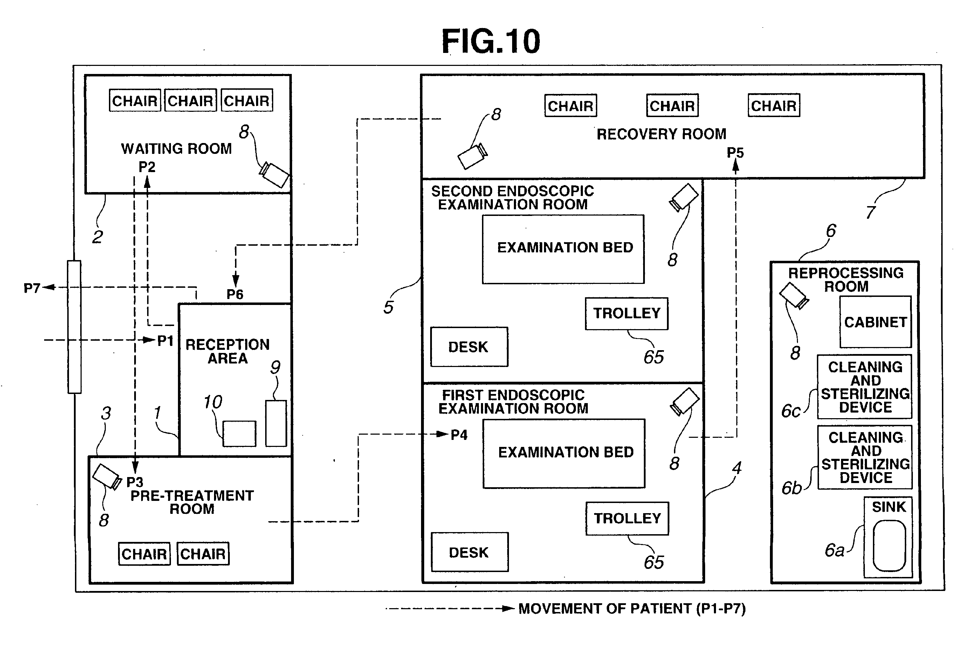

The action of the present embodiment having a composition of this kind will now be described. Firstly, the movements of a patient inside the hospital will be described with reference to Fig. 9 and Fig. 10. The movements of a patient inside the hospital are indicated by symbols Pl - P7 in Fig. 9 and Fig. 10.

As shown in Fig. 9, in this sequence of movements performed by the patient, upon arriving at the hospital (step S1), the patient first carries out reception procedures (step S2) for his or her examination in the reception area 1, and then waits in turn for an examination in the waiting room 2 (step S3).

When his or her turn comes, the patient then undergoes preliminary treatment (step S4) in the pre-treatment room 3, prior to endoscopic examination. Here, preliminary treatment, such as administration of local anaesthetic, or the like, is carried out, and a wait is carried out until the anaesthetic takes effect (step S5). Thereupon, the patient goes to the endoscopic examination room 4, and receives an endoscopic examination (step S6). This endoscopic examination may involve a standard diagnosis procedure, or alternatively, depending on the details of the diagnosis, a biopsy or treatment procedure, or the like (step S7) may be carried out. When the examination has been completed in the endoscopic examination room 4 (step S8), the patient is transferred to a recovery room 7 to undergo recovery (step S9), in order to restore his or her physical condition after the examination. Thereupon, when the patient has recovered, account processing (step S10) is carried out in the reception area 1, and the patient then is discharged (step S11).

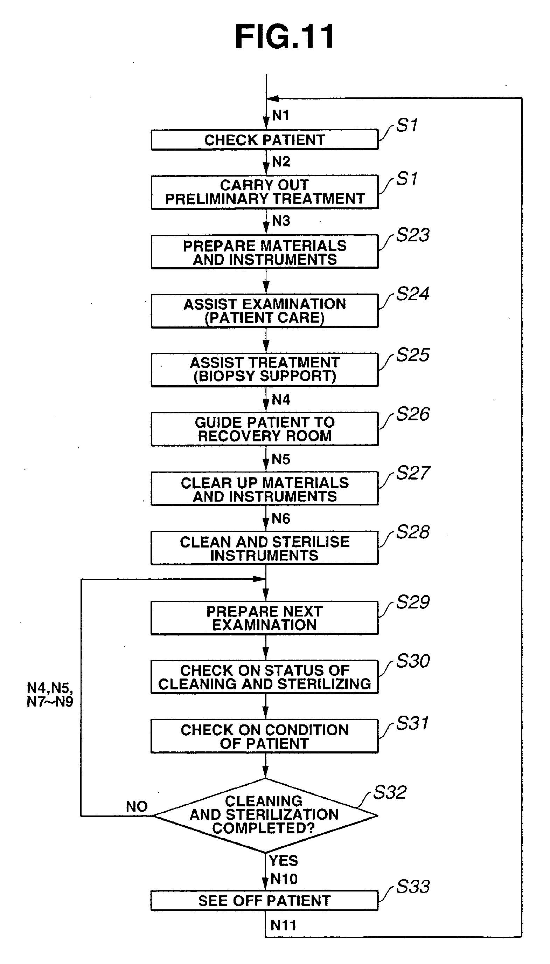

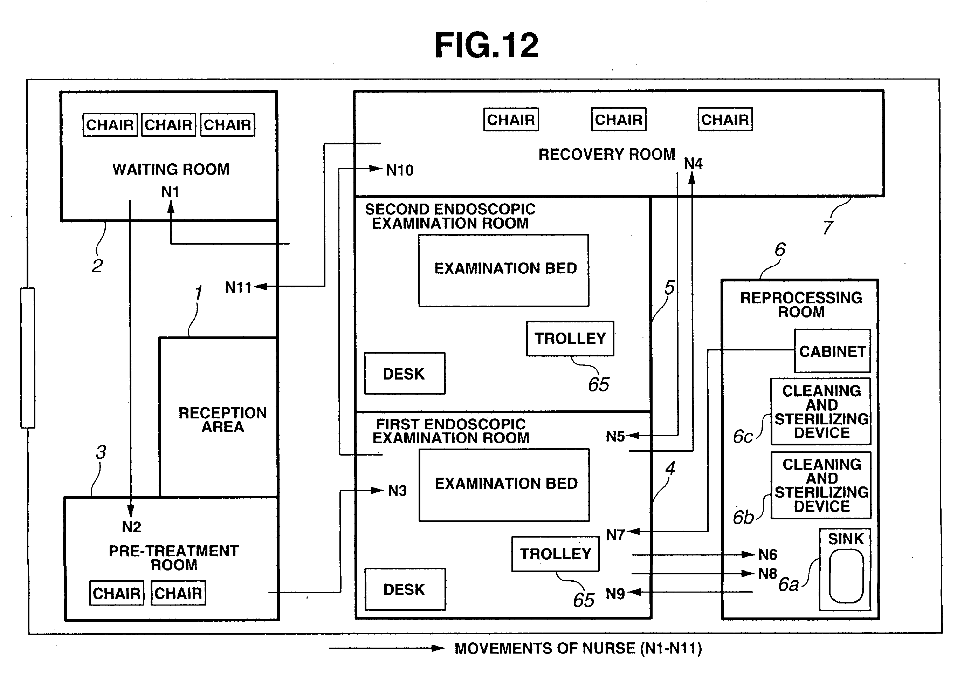

Next, the movements of a nurse in a conventional hospital, in other words, a hospital which does not have TV cameras 8 and portable terminals, will be described with reference to Fig. 11 and Fig. 12. The movements of the nurse in the hospital are indicated by symbols N1 ― N11 in Fig. 11 and Fig. 12.

As shown in Fig. 11, when a patient has completed reception procedures in the reception area 1, and is waiting in turn for his or her examination in the waiting room 2, then the nurse goes to the waiting room in order to verify the next patient to be treated, whilst also carrying out other tasks relating to the previous examination (step S21). Thereupon, the nurse takes the next patient in the order of treatment to the pre-treatment room 3, where the patient undergoes preliminary treatment prior to endoscopic examination (step S22). The nurse then goes to the endoscopic examination room 4 during the time that it takes for the anaesthetic to take effect in the patient, and the nurse performs other tasks (such as preparing materials and instruments for examination, and the like) (step S23).

When the preliminary treatment has been completed, the nurse leads the patient to the endoscopic examination room 4, lays the patient down on the bed, and prepares the endoscope and treatment instruments for endoscopic examination. In practical terms, the nurse sets up the endoscopic examination monitor 63, the CCU 61 and the light source unit 62.

When the endoscopic examination is to begin, the nurse performs tasks that are auxiliary to the examination (steps S24, S25), such as controlling the illumination in the examination room, handling treatment instruments, recovering tissue after a biopsy, and caring for the patient.

When the inspection in the endoscopic examination room 4 has been completed, the nurse leads the patient to the recovery room 7 in order to restore his or her physical condition after the examination (step S26), and the patient then undergoes recovery. Thereupon, the nurse goes from the recovery room 7 to the endoscopic examination room 4, and tidies up the materials and instruments used in the examination (step S27: simple cleaning of the endoscope may also be carried out at this stage). In order to carry out time-consuming reprocessing of the endoscope (namely, cleaning and sterilisation processing), the nurse transports (moves) the video endoscope, treatment instruments, and other peripheral items required for reprocessing, from the endoscopic examination room 4 to the reprocessing room 6, and carries out reprocessing (step S28).

Reprocessing generally comprises the following steps (see Fig. 12). Firstly, the endoscope is immersed in a sink 6a filled with water, air is injected into the main body of the endoscope, and it is confirmed that the pinhole (aperture) in the endoscope is not opened. If the pin hole (aperture) in the main body of the endoscope is open when the endoscope is being cleaned and sterilised, then the endoscope can become damaged by water infiltrating inside the endoscope, and hence the pinhole must be checked before carrying out these operations. When this task has been completed, the endoscope is placed in a cleaning and sterilising device 6b (6c).

During the time that the endoscope is being processed in the cleaning and sterilising device 6b (6c) (approximately 20 to 30 minutes), the nurse goes to the endoscopic examination room 4 in order to prepare the materials and instruments for the next examination, and if the cleaning and sterilising device 6b (6c) is still operating, then he or she goes to the recovery room 7 and checks up on the condition of the patients who have completed examination (steps S29 - S32).

This point in the work flow is a dynamic one and is the work period where the nurse is most occupied. In other words, the nurse must make preparations for the next examination whilst checking up on the condition of the patients, and confirming the operational state of the cleaning and sterilising device (checking that it is working properly, and whether or not it has completed operation, and the like). It is standard practice that the same endoscope used in the previous examination be used in the next examination, and therefore the operational state of the cleaning and sterilising device is very important, and if any problem occurs, the nurse must procure a replacement endoscope. When these tasks have been completed, the nurse goes to the recovery room 7, and takes a patient who has completed their examination to the reception area 1 (step S33). The nurse then returns to the waiting room 2 to check on the next patient (awaiting their turn for examination) (step S21).

As described above, it can be seen that, in a conventional hospital, the movements of a nurse from before examination until after examination are very complicated and require the nurse to be highly occupied and input a great deal of effort.

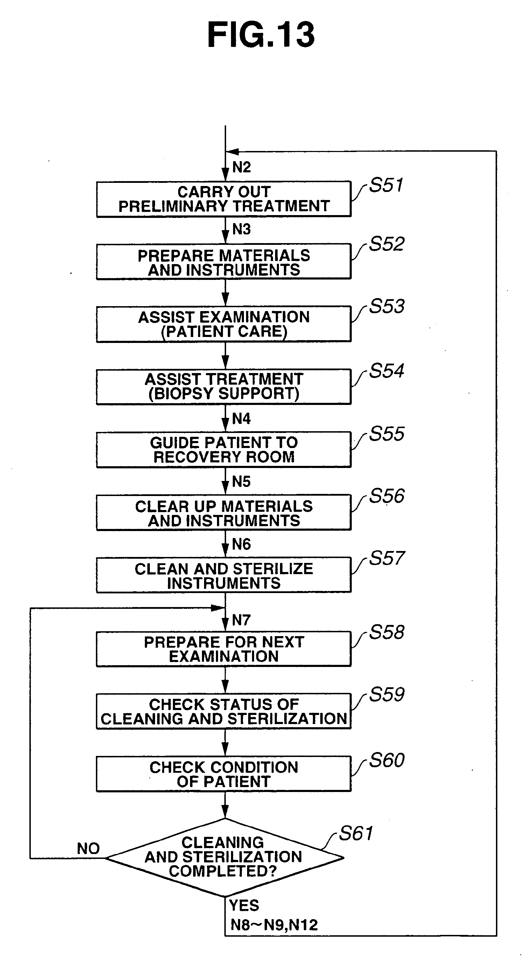

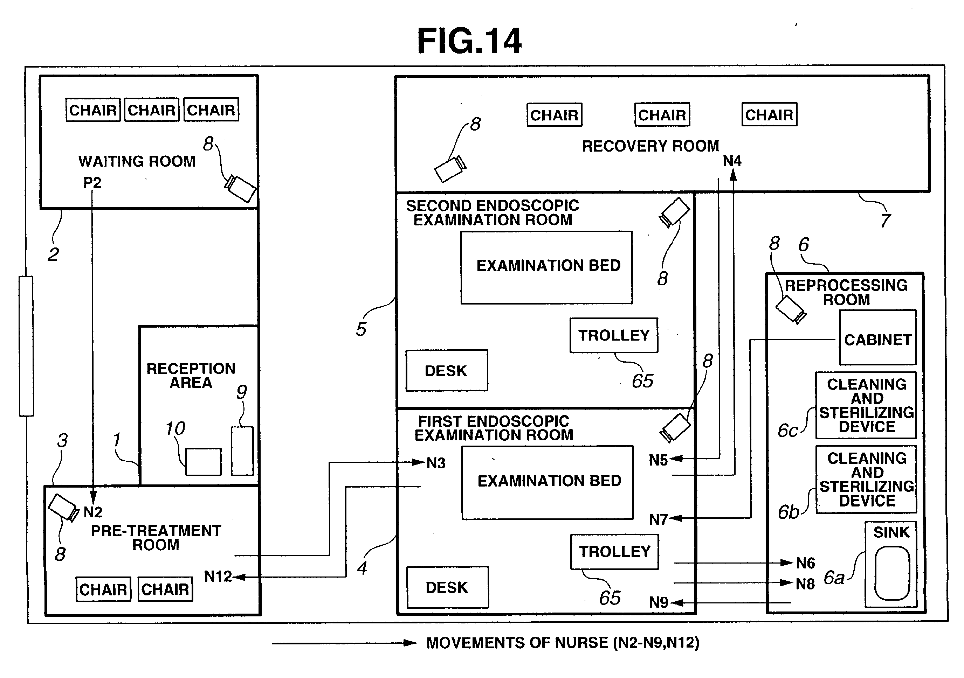

Next, the movements of a nurse in a hospital which has introduced the nurse support system according to the present embodiment illustrated in Fig. 8 will be described with reference to Fig. 13 and Fig. 14. The movements of the nurse in this hospital are indicated by the symbols N2 - N9, N12 in Fig. 13 and Fig. 14.

Firstly, the patient completes reception procedures for his or her examination in the reception area 1, and then awaits his or her turn in the waiting room 2. The nurse, whilst performing various tasks relating to the previous examination, operates the camera image switch SW24 of the portable terminal 11 in order to confirm the name of the next patient in the queue, and he or she checks on the number of patients waiting in the waiting room 2. Moreover, at the same time, the nurse checks on the number of patients waiting in the pre-treatment room 3 (this nurse is actually working in a location apart from the waiting room 2). In this case, if it is necessary to guide a patient somewhere, or to perform preliminary treatment, then using the call function on the portable terminal 11, the nurse can make contact with reception, or with another nurse, and ask them to guide the patient or carry out the preliminary treatment (step S51). Consequently, the nurse in question can carry on with the task in hand, without having to interrupt their current task and go to another location.

Next, when the other tasks have been completed (preparation of materials and instruments for examination, and the like: step S52), the nurse guides patients who have completed preliminary treatment to the endoscopic examination room 4.

Thereupon, the nurse lays the patient down on a bed, prepares the endoscope and treatment instruments for the endoscopic examination, and sets up the endoscopic examination monitor 63, the CCU 61 and the light source unit 62. Here, at the moment that the examination ends, the nurse can simultaneously brighten the illumination in the endoscopic examination room 4, by operating the remote control function of the portable terminal 11 (in the prior art, the nurse has to move across the endoscopic examination room 4 to operate the light switch, but here, the nurse can operate this function without moving).

Next, as auxiliary tasks to assist the examination (steps S53, S54), the nurse handles the treatment instruments, recovers tissue after a biopsy has been taken, and also cares for the patient. When the examination has been completed in the endoscopic examination room 4, the nurse leads the patient to the recovery room 7 (step S55) in order that the patient can recover his or her physical condition after the examination, and the patient undergoes recovery.

Thereupon, the nurse moves from the recovery room 7 to the endoscopic examination room 4, and clears away the materials and instruments used in the examination (step S56: simple cleaning of the endoscope may also be carried out at this stage). Here, in order to carry out reprocessing of the endoscope (namely, cleaning and sterilisation processing), the nurse transports the endoscope, treatment instruments, and other peripheral items required for reprocessing, from the endoscopic examination room 4 to the reprocessing room 6, and carries out reprocessing (step S57). He or she checks that the pinhole (aperture) in the main body of the endoscope is not open, and then places the endoscope in a cleaning and sterilising device 6b (6c).

During the time that the endoscope is being processed in the cleaning and sterilising device 6b (6c) (approximately 20 to 30 minutes), the nurse goes to the endoscopic examination room 4 and prepares the materials and instruments for the next examination. Here, by operating the camera image switch 24 on the portable terminal 11, the nurse checks the number of patients waiting in the waiting room 2, whilst also checking the number of patients waiting in the pre-treatment room 3, the progress made in the other endoscopic examination room 5, and whether or not the patient in the recovery room 7 has made a recovery yet. Here, if the nurse cannot interrupt his or her own work, then by using the call function of the portable terminal 11, the nurse can contact another nurse, and ask him or her to guide a patient somewhere, or to carry out preliminary treatment, or to prepare the patient to be discharged. However, if the nurse can handle these tasks him or herself, then he or she will do so (steps S58 - S61). Consequently, the nurse is able to continue with a task in hand, without having to interrupt their current work and move to another location.

Moreover, since the nurse can check whether or not the cleaning and sterilising device 6b (6c) in the reprocessing room 6 is operating correctly, by controlling the camera image switch 24 on the portable terminal 11, whilst performing other tasks, then the nurse can deal with any trouble that arises straight away, thereby preventing time losses.

Since the nurse is able to identify the conditions of the patients in the hospital, and the operational state of the cleaning and sterilising device 6b (6c), by means of the portable terminal 11, then he or she can continue with the task in hand, such as guiding a patient, or the like, without having to interrupt the current work. Consequently, the nurse can go from the endoscopic examination room 4 to the pre-treatment room 3 in order to undertake the action N12 of dealing with the next patient.

According to the present embodiment described above, it is possible to reduce the movements made by nurses within the hospital, and it is possible to support the work of nursing staff in such a manner that they can carry out their work in a highly efficient manner.

### Second embodiment

The second embodiment of the present invention is virtually the same as the first embodiment, and hence only the points of difference will be described here, and any similar elements of the composition are similarly labelled and description thereof is omitted.

As illustrated in Fig. 15, the present embodiment comprises, in place of the TV cameras 8, a host computer 100 installed in the reception area 1, for confirming the reception status of patients and the status of appointments, for example (a host computer comprising a server, PC, and terminal) 100; as well as terminal PCs 101 - 105 situated in a waiting room 2, pre-treatment room 3, endoscopic examination rooms 4, 5, recovery room 7; cleaning and sterilising devices 106, 107 having communications means situated in a reprocessing room 6; and portable terminals 11 (see Fig. 16) which communicate various information with the host computer 100, via signal communications means in the host computer 100.

In the present embodiment, by managing the terminal PCs 101 - 105, the cleaning and sterilising devices 106, 107, and the information communicated with the portable terminal 11, by means of the host computer 100, then a medical information system for assisting work involved in endoscopic examination, confirming the status of endoscope reprocessing, managing the stock of consumables of various types used in endoscopic examination, and the like, is constituted by the host computer 100.

As shown in Fig. 16, the host computer 100 and the terminal PCs 101 - 105, and the host computer 100 and the cleaning and sterilising devices 106, 107, are connected by means of a wired LAN network or a wireless LAN network. Moreover, the host computer 100 and the portable terminals 11 are connected by means of a wireless LAN. The communications means are not restricted in particular, and the present embodiment can be implemented by using any suitable existing communications means.

By means of the terminal PCs 101 - 105, at the reception area 1, appointment information and reception information is entered, whilst in the pre-treatment room 3, the application or non-application of preliminary treatment for the patient, and stock information regarding consumables, are input, in the endoscopic examination rooms 4, 5, examination start and end records are created and diagnostic images are filed, and the like, whilst in the recovery room 7, the patient is able to input recovery results relating to him or herself.

The medical information management system described above provides integrated management for managing the status of patient appointments, creating and managing reports relating to endoscopic examinations, storing images from endoscopic examinations, managing reprocessing information relating to the endoscope cleaning and sterilising devices (for example, the operational status of the equipment, the history of the reprocessing operation, management data regarding the consumables used in the equipment), and managing the stock of consumables used in endoscopic examination, and the like.

Medical information management systems of this kind already exist, and the characteristic feature of the present embodiment is that by adding the functions of the portable terminals 11 to the system, it is possible, in particular, to reduce unnecessary movements and actions performed by the nurses, and thus to improve the efficiency of the daily work undertaken by nursing staff.

In the first embodiment, it was sought to improve work efficiency by allowing the nurse to access information from the various rooms by confirming images captured by TV cameras 8, on his or her portable terminal 11, by operating the camera image switch 24, but in the present embodiment, it is sought to improve work efficiency by allowing the nurse to download information from the terminal PCs 101 - 105 and information from the cleaning and sterilising devices 106, 107, to his or her own portable terminal 11, via the host computer 100, by operating a data download switch 28 so that the nurse confirms the information on his or her portable terminal 11. In practical terms, for example, a patient completes reception procedures for an examination at the reception area 1, and then waits in turn for his or her examination in the waiting room 2. The nurse, whilst carrying out various tasks relating to the previous examination, checks the appointment status for endoscopic examinations in the host computer 100 in the reception area 1, by operating the data download switch 28 on the portable terminal 11, in order to check the name of the next person to receive an examination. Moreover, at the same time, the nurse can also check, via the host computer 100, information on the number of people who have completed preliminary treatment, as input via the terminal PC 102 in the pre-treatment room 3.

Moreover, at steps S53 and S54 described in Fig. 13 in relation to the first embodiment, the nurse handles the treatment instruments, recovers tissue after a biopsy has been taken, and cares for the patient, but if the nurse uses any consumables, or the like, during this, then he or she inputs this information by means of the data recognition function (RF-ID reader) of the portable terminal 11.

Furthermore, in the steps S58 to S61 described in Fig. 13, by accessing the information in the host computer 100 input via the terminal PC 101 in the waiting room 2, the terminal PC 102 in the pre-treatment room 3, or the terminal PCs 103, 104 in the endoscopic examination rooms 4, 5, by operating the data download switch 28 on the portable terminal, the nurse is able to check how many patients are waiting in the waiting room 2, how many patients are waiting in the pre-treatment room 3, what progress has been made in the other endoscopic examination room (a doctor may also identify the state of progress by operating the terminal PCs 103, 104), and whether or not the patient or patients in the recovery room 7 have recovered. Moreover, here, by operating the data download switch 28, the nurse can also obtain information relating to the cleaning and sterilising devices 106, 107, to check whether or not the cleaning and sterilising devices 106, 107 are operating correctly.

The rest of the action of this embodiment is the same as that of the first embodiment.

In this way, in the present embodiment, it is possible to obtain similar beneficial effects to those of the first embodiment.

### Third Embodiment

The third embodiment of the present invention is virtually the same as the second embodiment, and hence only the points of difference will be described here, and any similar elements of the composition are similarly labelled and description thereof is omitted.

In this embodiment, as illustrated in Fig. 17, in addition to a host computer 100, terminal PCs 101 - 105, cleaning and sterilising devices 106, 107, and nurse's portable terminals 11 which are carried by the nurses, there are also provided: patient's portable terminals 201 which are carried by patients, doctor's portable terminals 202 which are carried by doctors, endoscopic examination room status indicator boards 203 (see Fig. 18) for displaying information indicating the statuses of the endoscopic examination rooms 4, 5, and endoscopic examination room information input devices 204 (see Fig. 19) for inputting the status of the endoscopic examination rooms 4, 5, and as shown in Fig. 20, the endoscopic examination room status indicator boards 203 and the endoscopic examination room information input devices 204 are situated in the endoscopic examination rooms 4, 5.

The nurse's portable terminals 11, patient's portable terminals 201, and doctor's portable terminals 202 have functions whereby voice communications can be conducted mutually between the terminals. Moreover, they also have a function for sending and receiving information to and from the host computer 100.

In endoscopic examination, there are a plurality of endoscopic examination rooms 4, 5, and examinations are carried out in a parallel fashion by a plurality of doctors in a plurality of examination rooms 4, 5. An endoscopic examination room information input devices 204 is situated in each of the plurality of examination rooms 4, 5, in such a manner that information can be input indicating whether an examination is in preparation, or has completed preparation, or whether an examination is in progress or has completed. Moreover, each endoscopic examination room information input devices 204 has a function for communicating with the host computer 100 and can send the input examination room information to the host computer 100. Furthermore, each endoscopic examination room information input devices 204 has a function for receiving information on the next patient from the host computer 100, as well as a function for displaying the information on the next patient thus received.

An endoscopic examination room status indicator board 203 is installed in one or more positions in the examination rooms 4, 5, and by displaying information on the status of each endoscopic examination room, as sent by the host computer 100, it is possible for doctors, patients and nurses readily to tell the current status in each of the examination rooms 4, 5.

Endoscopic examinations are carried out in parallel by a plurality of doctors in a plurality of examination rooms 4, 5. When one examination has ended, the doctor inputs the end of the examination at an endoscopic examination room information input device 204. The examination end information is transmitted to the host computer 100, and the host computer 100 causes the endoscopic examination room status indicator board 203 to display the end of the examination.

Furthermore, at the same time, the end of the examination is indicated on the nurse's portable terminals 11. Thereby, a nurse is able to confirm that an examination has ended, by means of the endoscopic examination room status indicator board 203 or the portable terminal 11, even if he or she is away from the examination room, and is able to proceed with clearing up tasks for the examination room where the examination has ended, straight away. Thereupon, when the nurse has started to clear up the room, the nurse inputs an in preparation message to the endoscopic examination room information input device 204.

Furthermore, the host computer 100 also causes the endoscopic examination room information input device 204 of the room where examination has ended to display patient information and required materials and instruments for the next examination to be carried out, on the basis of a previously determined endoscopic examination schedule for the day in question. On the basis of this information, when the nurse has finished clearing up, he or she prepares the examination materials and instruments required for the next patient, in accordance with their particular condition.

Moreover, the host computer 100 also communicates with the patient's portable terminal 201 of the next patient, indicating to him or her that the examination will start forthwith, as well as the examination location, namely examination room 4 or 5, and the like.

Having completed preparations in the examination room 4, 5, the nurse inputs preparation completed information to the endoscopic examination room information input device 204. The preparation completed information is transmitted to the host computer 100, and the host computer 100 then causes a preparation completed message to be displayed on the endoscopic examination room status indicator boards 203. Simultaneously with this, a preparation completed message is displayed on the doctor's portable terminals. Thereby, the doctors are able to confirm that preparation has been completed, by means of an endoscopic examination room status indicator board 203 or a doctor's portable terminal 202, even if they are away from the examination room 4, 5, and hence they can make their way to the endoscopic examination room.

At the same time, the host computer 100 displays an examination preparation completed message on the patient's portable terminal 201, and can instruct the patient to make his or her way to the examination room 4 or 5. Upon receiving this instruction, the patient proceeds from the pre-treatment room 3 to the endoscopic examination room 4 or 5.

When the examination is to be started in endoscopic examination room 4 or 5, then examination in progress information is input to the endoscopic examination room information input device 204. This information is transmitted to the host computer 100, and the host computer 100 then causes the endoscopic examination room status indicator boards 203 to display an examination in progress message. Simultaneously with this, an examination in progress message is displayed on the nurse's portable terminals 11.

Furthermore, the endoscope, treatment instruments, peripheral items, and the like, used in the examination are cleaned and sterilised in respective cleaning and sterilising devices 106, 107. When the cleaning and sterilising devices 106, 107 start processing, they report a start of processing message to the host computer 100, via communications means. When processing is completed, they report an end of reprocessing message to the host computer 100. Alternatively, if an abnormality has arisen during processing, then a message to this effect is reported to the host computer 100. In the host computer 100, operating information is reported by the cleaning and sterilising devices 106, 107, and the operating information for the devices is displayed on the endoscopic examination room status indicator boards 203, and on the nurse's portable terminals 11. Thereby, as soon as reprocessing has finished, or as soon as an abnormality has arisen, the nurse is able to receive this information, and hence he or she does not have to go specifically to check on the operating statuses of the cleaning and sterilising devices 106, 107.

In the cleaning and sterilising devices 106, 107, the state of consumption of consumable items, such as sterilising solutions, detergents, filters, and the like, is transmitted to the host computer 100, and when the time for replacement is near, a message to this effect is sent from the host computer 100 for display on the nurse's portable terminals 11, in such a manner that consumables can be replaced at appropriate times.

Moreover, the respective portable terminals 11, 201, 202, 203 each has functions permitting voice communications between the terminals, and hence a doctor or nurse is able to check on the status of a patient who is waiting in pre-treatment or recovery, for example, without having to go directly to check in person. Furthermore, if a patient is feeling unwell, then he or she can let the doctor or nurse know successively his condition is not good, by means of the portable terminal 201. Moreover, calls and instructions between doctors and nurses can also be handled in succession by means of the terminals.

In the embodiments described above, by means of the host computer 100 identifying the examination status in an endoscopic examination room 4 or 5, and the operational status of the equipment, these statuses are monitored and information can be selected successively in such a manner that information required respectively by doctors, patients and nurses, can be transmitted respectively to same, in a selective fashion. Thereby, even without checking directly on the respective statuses by going in person to the various different areas involved, a nurse or doctor is able to carry out his or her work in a highly efficient manner, simply by proceeding to the point where they are required, when they are required at that location.

The examination schedule and state of progress are checked by the host computer 100, and by transmitting waiting time information to the patient, or informing the nurses or doctors immediately by means of the terminals if a patient is feeling unwell, then it is possible to provide satisfactory patient care.

In this invention, it is naturally possible to compose various different modes of implementation covering a broad range of applications, on the basis of the present invention, without departing from the spirit or scope of the invention. This invention is limited by the accompanying claims, but apart from this, it is not limited by the specific embodiments described herein.

### Industrial Applicability

As described above, the nursing work support system according to the present invention is valuable as a system for an industry where it is sought to improve the work efficiency of workers in service work, for example (customer service work, or the like), where a plurality of complicated work tasks arise simultaneously.

## Claims

1. A nursing work support system for supporting the work of nurses employed in medical examinations, who move between a plurality of medical work areas, comprising:
information obtaining means for obtaining area information relating to the plurality of medical work areas; and
portable terminal means carried by a nurse for communicating with the information obtaining means and acquiring area information relating to the plurality of medical work areas obtained by the information obtaining means.

2. The nursing work support system according to claim 1, wherein the information obtaining means comprises:
area image pickup means for capturing images of the medical work areas; and
information transmission means for transmitting image information captured by the area image pickup means, to the portable terminal means.

3. The nursing work support system according to claim 1, wherein the information obtaining means comprises:
a plurality of information input terminals disposed in the plurality of medical work areas; and
information managing means for managing information input by the plurality of information input terminals and transmitting same to the portable terminal means.

4. The nursing work support system according to any one of claims 1 , 2 or 3, wherein the plurality of medical work areas comprise at least an endoscopic examination room and an endoscope preparation room.

5. The nursing work support system according to claim 4, wherein the plurality of medical work areas comprise at least a patient pre-examination waiting room and a patient post-examination recovery room.

6. The nursing work support system according to claim 4, wherein a reprocessing device for cleaning and sterilizing endoscopes is provided in the endoscope preparation room, and the information obtaining means obtains at least information about the operation of the reprocessing device.

7. The nursing work support system according to claim 3, comprising area use status display means for displaying information about the use status of each of the plurality of medical work areas.

8. A nursing work support method for supporting the work of nurses employed in medical examinations, who move between a plurality of medical work areas, comprising:
an information obtaining step of obtaining area information relating to the plurality of medical work areas by information obtaining means; and
an area information display step of communicating with the information obtaining means and displaying the area information relating to the plurality of medical work areas obtained in the information obtaining step, on portable terminal means which can be carried by a nurse.

9. The nursing work support method according to claim 8, wherein the information obtaining step comprises:
an area image pickup step of capturing images of the medical work areas; and
an information transmitting step of transmitting image information captured in the area image pickup step, to the portable terminal means.

10. The nursing work support method according to claim 8, wherein the information obtaining step comprises:
an information managing step of managing information input by a plurality of information input terminals disposed in the plurality of medical work areas, and transmitting same to the portable terminal means.

11. The nursing work support method according to any one of claims 8, 9 or 10, wherein the plurality of medical work areas comprise at least an endoscopic examination room and an endoscope preparation room.

12. The nursing work support method according to claim 11, wherein the plurality of medical work areas comprise at least a patient pre-examination waiting room and a patient post-examination recovery room.

13. The nursing work support method according to claim 11, wherein a reprocessing device for cleaning and sterilizing endoscopes is provided in the endoscope preparation room, and the information obtaining method obtains at least information about the operation of the reprocessing device.

14. The nursing work support method according to claim 10, comprising an area use status display step of displaying information about the use status of each of the plurality of medical work areas.
